Europäisches Patentamt

European Patent Office

Office européen des brevets

(11) Publication number: **0 077 826**
**B1**

# EUROPEAN PATENT SPECIFICATION

(45) Date of publication of patent specification: **25.01.89**

(21) Application number: **82901777.1**

(22) Date of filing: **03.05.82**

(86) International application number:
**PCT/US82/00584**

(87) International publication number:
**WO 82/03770 11.11.82 Gazette 82/27**

(51) Int. Cl.⁴: **A 61 K 31/74,** A 61 K 31/745,
A 01 N 59/00, A 61 K 37/00

(54) AGENTS FOR ARRESTING FULMINATING INFECTION.

(30) Priority: **04.05.81 US 260144**

(43) Date of publication of application:
**04.05.83 Bulletin 83/18**

(45) Publication of the grant of the patent:
**25.01.89 Bulletin 89/04**

(84) Designated Contracting States:
**AT BE CH DE FR GB LI LU NL SE**

(56) References cited:
**DE-A-1 617 799**
**DE-A-2 652 088**
**FR-A-2 334 365**
**FR-M- 161**
**GB-A-2 020 551**
**US-A-1 920 492**
**US-A-2 312 715**
**US-A-2 581 035**
**US-A-2 728 732**
**US-A-3 095 410**
**US-A-3 864 473**
**US-A-3 922 342**
**US-A-4 029 770**
**US-A-4 061 735**
**US-A-4 137 307**
**US-A-4 174 277**
**US-A-4 248 685**

(73) Proprietor: **EVREKA, INC.**
**16 Foster Street**
**Bergenfield, NJ 07621 (US)**

(72) Inventor: **CERAMI, Anthony**
**76 Flanders-Drakestown Road**
**Flanders, NJ 07836 (US)**

(74) Representative: **Denmark, James**
**c/o Bailey Walsh & Co. 5 York Place**
**Leeds LS1 2SD Yorkshire (GB)**

# EP 0 077 826 B1

(56) References cited:

D. Falkenhagen et al., "Serum complement and protein metabolism in chronic dialysis patients", INT.J.ART.ORG. 2(2), at 65-68 (1979)

F. Wesenberg, "Characterization of heat eluates of human malignant tissues", ACTA PATH. MICROBIOL. SCAND., Sect. C, 87 at 301-306 (1979)

F. Archibald et al., "Removal of iron from human transferring by Neisseria Meningitidis" FEMS MICROBIOL. LETTERS 6 at 159-162 (1979)

G. Calver et al., "Inhibition of the growth of Neisseria Meningitidis by reduced ferritin and other iron-binding agents", INF, and IMM. 25 (3) at 880-890 (1979)

J. Davis et al., "A possible toxic factor in abdominal injury", J. TRAUMA, 2, 291-300 (1962)

Litwin et al., "Synergistic Toxicity of Gramnegative Bacteria and free colloid hemoglobin", ANN. SURG., 157 (4), 485-493 (1963)

T. Hau et al., SURGERY, 87 (5), 588-592 (1980)

J. Fletcher, "The effect of iron and transferrin on the killing of Escherichia coli and fresh serum", IMMUNOL., 20, 493-500 (1971)

A. Trippestad et al., "The role of hemoglobin for the lethal effect of intestine strangulation fluid", J. SURG. RES., 10 (10), 465-470 (1970)

J. Eisenlauer et al., "Stability of colloidal silica (aerosil)hydrosols, II. Influence of the pH value and the adsorption of polyethylene glycols", J. COLLOID AND INTERFACE SCI., 74(1), 120-135, (1980)

B.E. Hallayway et al., "Changes in conformation and function of hemoglobin & mycoglobin induced by adsorption to silica", BIOCHEM. BIOPHYS. RES. COMM., 86 (3), 689-696 (1979)

J. Eisenlauer et al., "Stability of Colloidal silica (Aerosil) hydrosols, I. Preparation and characterization of silica (Aerosil) hydrosols", J. COLLOID INTERFACE SCI., 74 (1), 108-119 (1980)

J.T. Lee, Jr. et al., SURGERY, 86 (1), 41-48, (1979)

J.H. Davis et al., "A toxic factor in abdominal injury II: The role of the red cell component", J. TRAUMA, 4, 84-90 (1964)

J.J. Bullen et al., "The effect of iron compounds on the virulence of Escherichia coli for Guinea-pigs", IMMUNOL., 15, 581-588 (1968)

G.H. Bornside et al., "Hemoglobin and Escherichia coli, a lethal intraperitoneal combination", J. BACT., 95 (5), 1567-1571 (1968)

T. Hau et al., SURGERY, 83 (2), 223-229, (1978)

## Description

Field of the invention

The present invention relates to animal tissue infections, and particularly to the use of agents for the manufacturing of medicaments useful in the control and inhibition of such infections.

Description of the prior art

The adverse pathological consequences of tissue trauma in humans and other animals, including the trauma induced by surgical procedures, has long been studied with a view to preventing the development of bacterial infections that as a rule do not respond well to antibiotic therapy. In particular, trauma and bleeding in the peritoneum of humans, such as results during abdominal surgery, appears to foster the rapid growth of bacteria that cause fulminating infections, that in the majority of cases, are fatal to the patient.

Experimental observations with animal models suggest that the hemoglobin in blood is responsible for an acceleration in bacterial growth. In particular, the simultaneous intraperitoneal administration of hemoglobin and the bacterium E. coli to mice resulted in a fulminating septicemia and rapid death that did not occur when either the bacteria or the hemoglobin were individually administered.

Efforts to reduce the occurrence and severity of fulminating bacterial infections, have been limited to the curtailment of unwanted bleeding and the physical removal of unwanted blood from the peritoneal area, in conjunction with the administration of antibiotic therapy. Despite such measures, bacterial infections that develop in this area continue to move rapidly out of control, and frequently are fatal to the patient.

Bacterial infections, and particularly those that develop at the site of tissue trauma, or at any location where bleeding takes place have long been of concern, and, as such, the subject of investigation, for the purpose of developing procedures for their control. Thus, whether topical or peritoneal in location, these bacterial infections frequently become fulminating in nature and can, in some instances result in death.

It is known to use certain agents to which the invention relates in animal therapy and examples of such use are disclosed in the following patent specifications:

DE—A—2652088
GB—A—2020551
FR—A—2334365
US—A—2581035
US—A—3922342
US—A—3922342
US—A—4137307
US—A—4061735

These specifications deal with silica for the most part and only generally refer to its use as a complexing agent however for various unrelated purposes. For example, GB—A—2020551 discloses the use of silica gel for the purpose of reducing the lipid concentration in the blood of a patient. Similarly, DE—A—2652088 discusses the use of silica in an appliance as illustrated in the figures, as an absorbent.

The US—A—4061735 and US—A—4137307 both disclose the use of haptoglobin as a treatment for renal disorders caused by hemolysis, the haptoglobin prepared in one aspect by heat treatment with a stabilising agent identified as hepatitis B virus.

Reference is also made to FEMS Microbiological Letters Volume 6, pages 159—162, 1929, to authors Archibald and DeVoe. This article concerns the removal of iron from human transferrin. As the disclosure indicates, the naturally occurring agents heptaglobin, transferrin and hemopexin possess the activity of being able to arrest infection by forming an indigestible complex with bioavailable iron. In the Archibald et al, article, the removal of iron from human transferrin was investigated, and a particular meningococcal strain was incubated with iron bearing transferrin, with the result that iron was removed from transferrin by the bacteria by a means not clearly identified, though suggested to be as a result of some high molecular weight transferrinase. This suggests that transferrin will not irreversibly bind to iron to form an indigestible complex.

In the article Immunology, at Volume 15, Pages 581—588 by Bullen, Leigh and Rogers, it is suggested that ferric ammonium citrate, haematin hydrochloride, guinea-pig red cells and hemoglobin iron containing compounds be administered to guinea-pigs which were previously innoculated with E. coli, to observe the course of the infection. The authors found that these iron containing compounds advanced death, and thereby confirm a long standing thesis that available iron at the location of infection enhances the rate of infection by providing nutrition to the organisms that cause the same. The authors conclude that transferrin plays a part in the resistance to infection, presumably by its capability of binding with iron. The authors however, do not identify what the role is that transferrin plays, or whether transferrin acts alone.

In fact, they suggest that transferrin acts in conjunction with other globulins, which are fractions of the blood plasma, to achieve whatever inhibitory result is observed.

To say that transferrin may play a role in resistance to infection is not to say that transferrin can arrest infection single handedly. Particularly when the Bullen article comes prior to the article by Archibald and DeVoe, it must be considered that the latter article is far more dispositive of the activities and capabilities of transferrin than the former. Thus, the earlier discussed article suggests that the role of transferrin could not include or contemplate the complete inhibition of infection, when the authors findings suggest that the bond between transferrin and iron can be broken.

In the article in Immunology, Volume 20, Pages 493—500 authored by Fletcher et al, there is disclosed an investigation of both iron and transferrin, which appears to deal primarily with the activity of iron in its unbound state. The authors conclude that iron appears to serve as both a growth factor and as a blocking agent to the bacteriocidal activity of serum, and in passing, acknowledges the status of transferrin as a binding agent. Their article, like Bullen et al, predates the Archibald article by roughly ten years and its findings must likewise be taken in the context of the later publication.

In the article in Infection and Immunology, Volume 25(3), Pages 880—890, authored by Calver, Kenny and Kushner, a study of meningococcus is made and it was found that both the virulent and avirulent strains could utilise either ionic or chelated iron. Inhibition of these organisms was considered with a variety of agents, including the naturally occurring iron binding proteins from animal fluids, such as transferrin. A specific comment with regard to transferrin was noted, as the authors concluded that transferrin was only effective to bind iron when used in conjunction with bicarbonate. Most of the work that was done by the authors was with ferritin and there was only speculation as to its effectiveness as an iron binding and thereby infection inhibiting agent.

The present invention concerns the use of various agents for the manufacture of medicaments for the treatment of bacterial infections in animals whereby said agents in use will form complexes inhibiting or eliminating bacterial growth. Although reference is made in the following to administering of the agents it will be appreciated that such agents are in the medicament form manufactured using the agents.

Summary of the invention

In accordance with the present invention there is provided the use of agents capable of aggregating red blood cells and/or hemoglobin selected from the complexing proteins haptoglobin; hemopexin; transferrin; a mixture of haptoglobin, hemopexin and transferrin; plant lectins specific for sugars, and mixtures thereof, colloidal silica, anion exchange resins, resins capable of binding compounds having available sulfhydryl groups, a composite of colloidal silica and polyolefinic elastomer, and mixtures thereof for the manufacture of a medicament for the treatment of bacterial infections.

Agents, the use of which is made in the present invention, include those materials that are capable of forming an immobilised complex with red blood cells as well as with hemoglobin. The agent may be used to make the complex with the red blood cells or the hemoglobin fraction of free blood in the vicinity of a tissue trauma to make the new tissue unavailable to infection-promoting bacteria, so that the bacteria cannot abstract the iron necessary for their growth. A wide variety of bacteria thrive on available iron, and range in activity from lethal, infection-causing varieties, to those that cause the evolution of undesirable odor during their life cycle.

The agents used in the present invention may include cellulose-based resins, and oxygenous haptoglobin. Preferably, the agent utilised comprises colloidal silica suspended in a matrix of polyisobutylene.

In the instance where the agent is administered to a peritoneal tissue trauma, and the source of iron may include hemoglobin, as well as heme or other iron salts, the agent may include haptoglobin in combination with other proteins, such as transferrin or hemopexin, individually and in mixtures with each other.

In the instance where the agent is administered to a peritoneal trauma, a sterile solution containing the agent may be prepared in a concentration that may range from 1.0 to 100 mg./ml. Administration may be either by direct dosage or by incorporation into conventional irrigation fluids. In the instance where topical treatments are contemplated, the agents must be disposed on the topical application or carrier in a manner such that the agent is available for surface contact with the blood or hemoglobin.

The various agents useful in the therapeutic application are widely available and are inexpensive to recover and utilise.

The complexing proteins may either be derived from human plasma, or may be synthetically prepared. Such complexing proteins are universally compatible with animal tissue and animal body fluids, so that the risk of adverse reactions to their administration is non-existent.

Accordingly it is a principal object of the present invention to provide a means for arresting in a radical and complete fashion bacterial infections that develop in the presence of body fluids offering available iron.

It is a yet further object of the present invention to identify a variety of agents for producing complexes usable in both topical and intraperitoneal applications.

Other objects and advantages will become apparent to those skilled in the art from a consideration of the ensuing description which proceeds with reference to the following illustrative drawings.

Brief description of drawings

Figures 1 and 2 graphically represent the results of in vitro comparative testing of agents used in the present invention.

Figures 3 and 4 graphically represent the results of in vitro comparative testing of agents used in the present invention.

Figures 5 and 6 graphically represent the results of in vitro comparative testing of colloidal silica used in the present invention.

Detailed description

In accordance with the present invention an agent is used to provide a medicament for producing a complex for arresting tissue infection in the presence of a body fluid containing iron accessible to the infection-promoting bacteria. The agent is administered to the locus of infection and is such as to produce a complex for providing that the iron is made nitritionally unavailable to the bacteria. In the instance where the body fluid is blood, the agent is adminstered in an amount sufficient to form a complex immobilising either the red blood cells or the hemoglobin present in the blood adjacent the bacteria. The amount of the agent administered which is significant will therefore be proportional to the amount of the component of the blood sought to be immobilised and herein lies novelty of application not heretofore suggested.

Agents according to the invention for producing the complex useful in the immobilisation of blood may be divided into two categories, depending upon the component of the blood for which immobilisation is desired. In the instance where it is desired to immobilise the red blood cell, the agents may comprise antibodies against the red blood cell, colloidal silica, anion resins and plant lectins capable of binding sugars, such as Concanavalin A. In the instance where the component to be immobilised is hemoglobin, the agent may be selected from antibodies against hemoglobin, colloidal silica, anion exchange resins and complexing proteins such as haptoglobin.

As discussed earlier, work previously done on this subject, has determined that bacteria were capable of digesting the red blood cell and hemoglobin, to abstract and absorb the contained iron, to make it available for nutrition and growth. In particular, Eaton et al, "Haptoglobin: A Natural Bacteriostat", Science, 215:691—692 (1982), summarised work performed on behalf and under the direction of the applicant and concerning the present invention, and restated the observations by earlier workers that the available iron abstracted from blood present in a wound, gave the bacteria sufficient nutrition to achieve rapid replication, so that the natural body defences were inadequate to control the resulting fulminating infection.

The present invention relates to the provision of a variety of agents to be utilised both peritoneally and topically, in amounts sufficient to bind either -available red blood cells or available hemoglobin to prevent either of the two from being broken down by the undesired bacteria for the purpose of abstracting the iron contained therein.

Under normal conditions, the iron-containing fractions of blood are maintained secure against breakdown by association with certain complexing proteins. In particular, the fraction hemoglobin is secured by association with the protein haptoglobin, while the heme fraction is bound by the protein hemopexin, and iron occurring as iron salts is bound by the protein transferrin. Each of the foregoing binding or complexing proteins, however, is present in relatively small amounts under normal circumstances. Increases of the levels of these proteins can occur gradually by normal body functions, in response to the onset of certain internal pathological conditions; however, the body is not equipped to rapidly provide the relatively massive amounts of these proteins necessary to maintain iron in the unavailable sequestered state, such as in the instance where tissue trauma takes place and the blood is released from the vascular system into the peritoneum and thereby into contact with infection-promoting bacteria. Similarly, in the instance where natural release of blood takes place, or in the instance of topical abrasions and cuts, the quantity of blood released is frequently far in excess of the capability of these natural complexing agents. The difficulty engendered by this problem is apparent from a consideration that the red blood cells derived from 1 milliliter of blood can yield up to 150 milligrams of hemaglobin after red cell breakdown, a value that far exceeds the capacity of any haptoglobin normally present in that quantity of blood.

In the instance where the agent comprises a complexing protein derived in exogenous form it is preferably administered in a sterile solution. The quantity of agent may naturally vary, in relation to the nature and extent of body fluid liberated by the trauma. As the proteins haptoglobin, hemopexin and transferrin exist in human blood in amounts of approximately 1.2 mg/ml, the quantities of such materials administered in accordance with the present method exceeds these amount substantially and herein lies the novel therapeutic method, and generally varies in relation to the amount of body fluid released by the trauma, in a ratio with respect thereto ranging up to 3 to 1. The ratio of the amount of agent administered with respect to body fluid released may preferably range from 2 to 1 to 3 to 1. Naturally, the foregoing proportions are provided herein as illustrations only, and in instances therefore where iron content may vary, the proportion of the agent administered with respect to body fluid released, may likewise differ, provided that the quantity administered is sufficient to render the iron nutritionally unavailable to the organisms.

The agent is preferably utilised in conjunction with conventional antibiotic therapy, and provides the advantage of preventing the acceleration of bacterial growth that has rendered such therapy inadequate in the past. Bacterial growth generally occurs slowly during the initial 24 hours of innoculation, during which time growth is characterised by a lag phase. After this period is surpassed, accelerated or logarithmic growth commences, and full infection of the tissues become pronounced and severe. It has been found that control of the spread of bacterial infection can be achieved if growth during this initial 24 hour period can be either restrained or reduced in rate.

Accordingly, the new therapeutic application proposes administering relatively large amounts of agents capable of binding either the red blood cells or the hemoglobin component of the blood released in any of these situations. Thus, in the instance of tissue trauma, due to surgical procedures or the like, a sterile solution containing the agent of the present invention may be directly administered by injection or otherwise, or may be incorporated in an irrigation fluid that is utilised to wash body wound areas. Additionally, the agent may be affixed to a surgical sponge which may then be utilised in contact with the blood. In the instance where the solutions of the agent are utilised, they may be prepared to concentrations of up to 100 milligrams per milliliter, and preferably from 1.0 to 100 milligrams per milliliter.

In the instance where the agent is utilised for topical application, it may be disposed on various topical appliances, such as bandages and gauze pads, as well as sanitary and catamenial devices such as tampons. For example, in this latter instance, the agent may be disposed within a container in a tampon or within the pores of the absorbent material, as disclosed, respectively, in US—A—3,842,166 to Bucalo and US—A—3,850,160 to Denson cited herein as exemplary only.

In the instance where the present agents comprise colloidal silica, a particular form of silica used for the colloidal silica comprises that known as "Cab-O-Sil®". This product is manufactured by the Cabot Corporation, Boston, Massachusetts, and is generally known for its use as an antiagglomerative in various compositions, and as a general absorbent. "Cab-O-Sil®" comprises submicroscopic, pyrogenic silica prepared in a hot, gaseous environment by a vapor phase flame hydrolysis, at high temperature (around 1100°C) of a silicon compound, such as silicon tetrachloride. It is distinct from silica gel obtained by precipitation of silicic acid from an aqueous silicone solution, and hardening of the precipitate. Silica gel, thus formed is internally porous, whereas "Cab-O-Sil®" has an enormous external surface area and no internal porosity.·

"Cab-O-Sil®" contains no water-soluble inorganic salts. It is of high chemical purity, low water content and has a high degree of particle separation. The properties and composition of a grade of "Cab-O-Sil®" are listed as follows:

Silica Content (moisture free)—99.0—99.7%
Free Moisture (105°C)—0.2—1.5%
Ignition loss at 1000°C (excluding moisture)—0.2—1.0%
CaO, MgO—0.00%
$Fe_2O_3+Al_2O_3$—0.01%
Particle size range—0.015—0.020 μm
Surface area—175—200 m²/g
Specific gravity—2.1
Colour—white
Refractive index—1.46
Apparent bulk density—40.05—112.14 kg/m³ (2.5—7.0 lbs/cu ft).
pH (when in a 4% colloidal silica aqueous dispersion form)—3.5—4.2

A finer grade of "Cab-O-Sil®" has the above characteristics but a particle size range of 0.007—0.010 μm, a surface area of substantially 325 m²/g, and a refractive index of 1.46.

As mentioned earlier, "Cab-O-Sil®" colloidal silica may be utilised alone in solution form as the agent. In the instance, however, where it is to be disposed either within or upon a carrier or substrate, it is preferably prepared in a composite particulate form with a polyolefinic elastomeric organic binder. In particular, food grade polyisobutylene may be utilised. In such instance, the colloidal silica is suspended in a dilute hydrocarbon solution of the elastomer, and the resulting suspension is spread on an appropriate surface and dried to form the flake-like particles of the composite.

In a preferred embodiment the composition of the colloidal silica and the polyisobutylene is prepared in proportions of approximately 85% colloidal silica, 15% polyisobutylene, such proportions determined by weight of the composite. This material may then be affixed by conventional procedures to a variety of substrates for further use.

In the instance where certain tests of the colloidal silica were made, where the colloidal silica was incorporated within a variety of fibrous materials which were then tested in contact with blood and bacteria on an in vitro basis, the materials ranged from rayon filament, to rayon staple, and fibres of various sizes, prepared from silica, silicic acid and fiber glass. In each instance, the colloidal silica was found to be inactive and infection proceeded unabated.

By contrast, the preparation of the colloidal silica-polyisobutylene composite offers the same activity as that of the colloidal silica alone, when disposed on, or within, a carrier or substrate.

Another class of agents, that is useful both for hemoglobin and red blood cells comprises anion exchange resins such as diethylaminoethylcellulose (DEAE-Cellulose), guanidoethylcellulose, diethyl (2-hydroxypropyl)aminoethyldextran (QAE-Sephadex®), and resins capable of binding compounds having available sulfhydryl groups, such as polyethyleneimine. The number of suitable anion exchangers is large, and the foregoing listing is merely exemplary.

Also, as mentioned earlier, various complexing proteins may be utilised as the agent, and in addition to haptoglobin and transferrin, complexing proteins such as the macromolecular binding materials known as plant lectins, may be utilised either in solution, or disposed on a substrate.

Lectins are known carbonhydrate-binding proteins derived from plants, having a wide variety of specificity. For example, Concanavalin A, a jack bean lectin, has specificity for α-D mannopyranose and α-D glucopyranose; soybean lectins show specificity for α and β - D - N - acetylgalactosamine and α - D - galactose units, while wheat germ lectin is specific for β - D - N - acetylglucosamine. Naturally, the specific complexing proteins selected and utilised will vary with the nature of the bacteria to be inhibited, as well as the conditions in which the bacteria are found.

Likewise, the agents of the present invention may be utilised inviddually or in mixture, depending upon the environment in which use is contemplated. For example, one may combine a quantity of DEAE-Cellulose with "Cab-O-Sil®" colloidal silica for use, for example, in the treatment of surgical wounds.

With respect to "Cab-O-Sil®" colloidal silica, the effective administration of this agent has been limited somewhat to instances where large amounts of free monovalent anion such as chloride are absent from the locus of administration. In such instances, the preparation of the composite "Cab-O-Sil®" colloidal silica - polyisobutylene particles appears to overcome this restriction, as such particles are capable of operating effectively even in the presence of physiological concentrations of chloride.

The operation of the agents of the invention, will be better understood from a consideration of the following examples, wherein amounts of materials expressed in percent, are intended to refer to percent by weight.

Example 1

A large number of synthetic growth media were prepared having a low iron content. The media were thereafter inoculated with equal quantities of the bacteria E. coli. Five primary groupings of inocula were developed as follows:

1. Group 1—The bacteria E. coli alone.
2. Group 2—E. coli and a small amount of hemoglobin (concentration 340 µg/ml).
3. Group 3—E. coli, hemoglobin, as in Group 2, above, and $1.5 \times 10^{-5}$ Molar concentration of haptoglobin purified from human plasma.
4. Group 4—E. coli, a small quantity of $10^{-5}$ Molar hemin and $2 \times 10^{-5}$ Molar concentration of haptoglobin.
5. Group 5—E. coli, a quantity of $1.5 \times 10^{-5}$ Molar concentration of haptoglobin alone.

In all of the above groupings, the volumetric quantities of hemoglobin, haptoglobin and hemin were roughly equivalent.

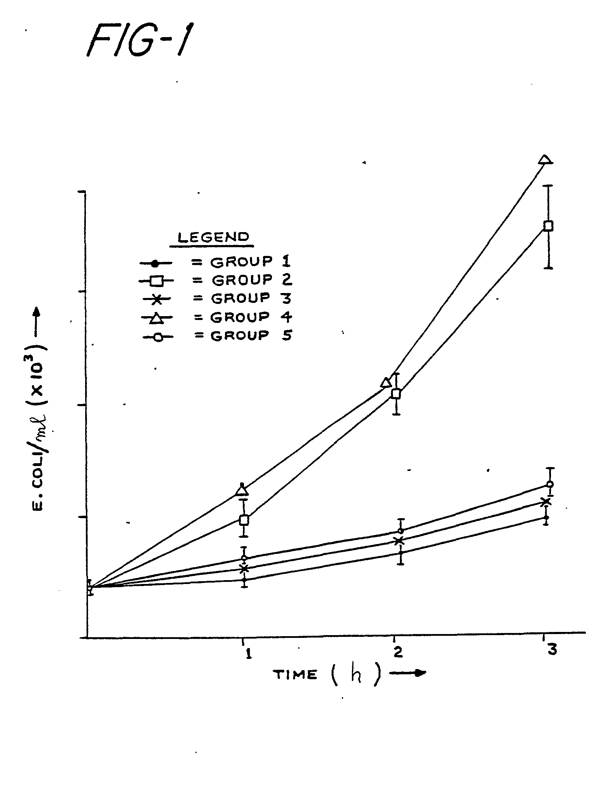

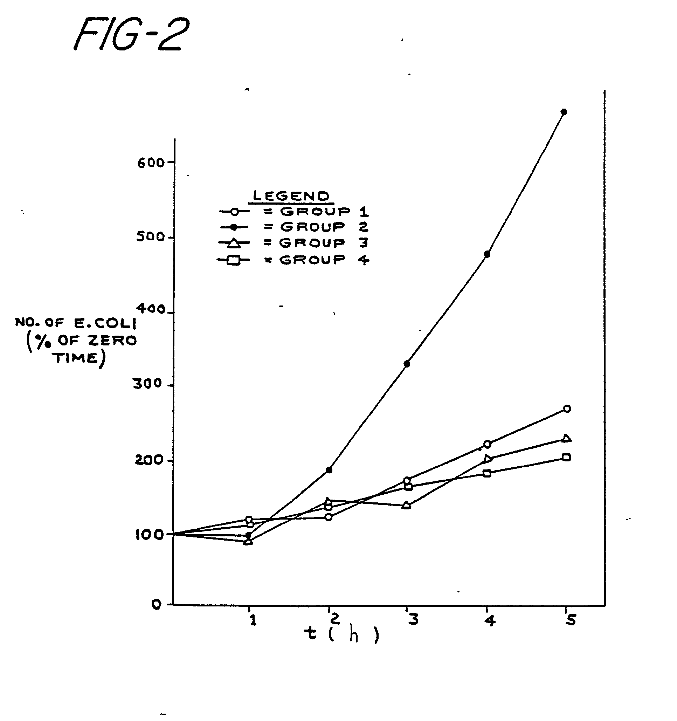

Two batteries of such tests were performed, the first of which included all five of the above groupings; the second battery included Groups 1—3 and 5 only. All samples were maintained at a temperature of 37°C, and were observed for a period of time ranging to about 24 hours following inoculation. At the end of the period, bacteria counts of the respective media samples were made, and the results for the first battery of tests were tabulated and set forth in Figure 1, while the test results for the second battery of tests are set forth in Figure 2.

The data from the respective groupings is identified by the corresponding graphical symbol set forth in the legends accompanying each Figure. The data points plotted in Figure 1 represent the respective mean and standard deviation values determined from a comparison of three samples of each groupings observed and counted at the indicated time interval.

Referring to Figure 1, the rate of growth of the bacteria E. coli, alone can be seen to be relatively flat, so that the initial or lag phase of bacterial growth is apparent. By contrast, the addition of the hemoglobin to the growth medium alone results in a domestic acceleration in bacterial growth. This growth, however, is neutralised in the instance of Group 3, wherein a comparable quantity of haptoglobin is introduced together with the hemoglobin, and is similar to rate to the growth pattern of the bacteria inoculated with haptoglobin alone.

The results of the Group 4 media illustrates the specificity of haptoglobin for hemoglobin. In these samples, haptoglobin was introduced, however hemin, containing a variant form of available iron was provided in place of hemoglobin. Haptoglobin proved ineffective in this instance, and greatly accelerated growth of the bacteria was in evidence.

The results of the Group 5 media show that the presence of bacteria and haptoglobin above has little effect on bacteria growth.

The data gathered from the first battery for tests was corroborated by further testing, shown in Figure 2. In similar fashion to Figure 1, the media containing the bacteria alone exhibited a relatively flat growth curve, while the media bearing the added hemoglobin exhibited a dramatic increase in the rate of bacterial growth within two hours.

The addition of a quantity of haptoglobin to the inoculum containing hemoglobin, shown in Group 3, appeared to neutralise the effects of the hemoglobin, so that the bacteria exhibited a growth rate that was, in fact, even flatter than the rate observed for the bacteria alone. Similarly, the growth rate for Group 5, where haptoglobin alone was added, proved flatter than that of the control Group 1.

The foregoing in vitro studies confirmed the specific effect that haptoglobin has upon bacterial growth in the presence of hemoglobin, and prompted the in vitro studies that are discussed below.

Example II

In vitro studies were conducted to determine the efficacy of the agents used. A total of 30 laboratory rats were divided into three groups, and were inoculated with intraperitoneal injections as set forth below.

1. Group 1—These rats were inoculated with $2 \times 10^{9}$ E. coli bacteria alone.
2. Group 2—These rats were injected with $2 \times 10^{9}$ E. coli together with 10 mg stroma-free hemoglobin.
3. Group 3—These rats received $2 \times 10^{9}$ E. coli, 10 mg stroma-free hemoglobin and 20 mg purified human haptoglobin (the quantity of haptoglobin estimated by its binding capacity).

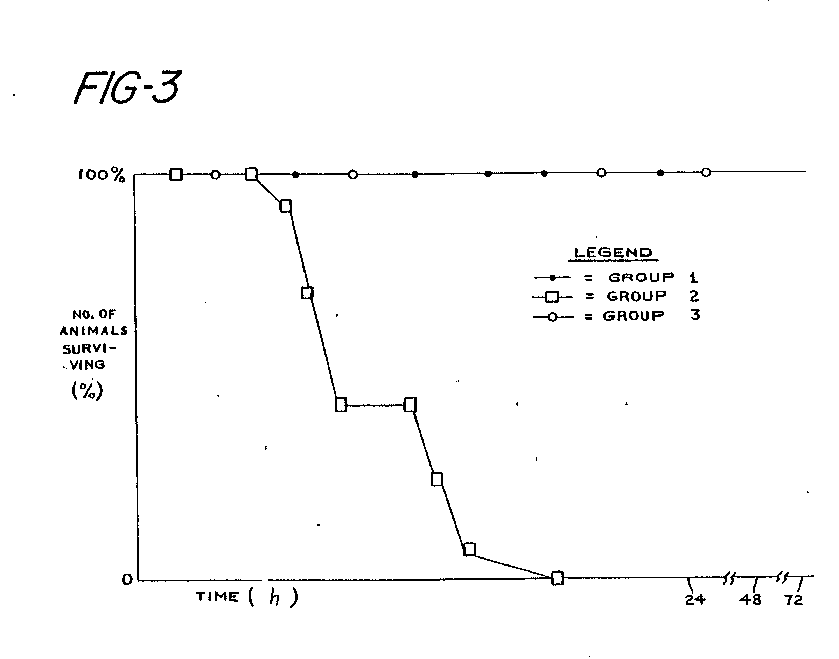

The bacteria utilised in this test was isolated from a fatal human case of septicemia. After injection, the rats were placed under observation for a period of 72 hours, to note the adverse effect, if any, caused by the respective injections. The rats were regularly observed during this period, and the results regarding survival were gathered and are presented in graphical form in Figure 3. The identity of the results of the respective groups may be determined from the legends accompanying the graph.

It is apparent from Figure 3, that the Group 1 subjects were able to successfully fend off the lethal effects of the bacteria alone, as administered in the Group 1 tests. By contrast, however, the introduction of hemoglobin to the inoculum, resulted in the death of all of the rats from overwhelming septicemia within 24 hours after injection.

The inclusion of haptoglobin in Group 3 appears to counteract the lethal effects of the hemoglobin, as all of the rats in this grouping survived the entire test period.

The results of this example illustrates that haptaglobin exerts a neutralizing effect upon hemoglobin in the presence of bacteria in an in vitro situation.

Example III

Additional in vitro testing was conducted with laboratory rats in a manner similar to that set forth in Example II above. Two batteries of tests were conducted, each battery differing only in the number of rats utilised, and the specific composition of the inocula.

In the first battery of tests, three groups each of seven male rats, each averaging in weight between 125 and 150 g, were injected with a 1.25 ml volume of individual preparations set forth according to group numbers, below.

1. Group 1—Inoculum comprised 20 mg stroma-free hemoglobin (SFHb) (3.4 g%), $2.5 \times 10^7$ E. coli bacteria and 66.7 mg Albumin.

2. Group 2—Inoculum comprised $2.5 \times 10^7$ E. coli and 86.7 mg Albumin, alone.

3. Group 3—Inoculum comprised $2.5 \times 10^7$ E. coli bacteria, 20 mg stroma-free hemoglobin (SFHb) (3.4 g%) and sufficient haptoglobin to bind 20 mg hemoglobin (HbBC=30 mg/ml) (equivalent to approximately 67 mg of protein).

The second battery of tests were performed with three groups of eight rats each, the rats otherwise identical to those utilised in the first battery of testing. The inocula utilised in the second battery of tests are set forth below.

1. Group 4—The inoculum comprised 20 mg stroma-free hemoglobin (SFHb) (3.4 g%), $2.5 \times 10^7$ E. coli bacteria and 66.7 mg Albumin.

2. Group 5—Inoculum comprised $2.5 \times 10^7$ E. coli and 86.6 mg Albumin, alone.

3. Group 6—Inoculum comprised $2.5 \times 10^7$ E. coli bacteria, 20 mg stroma-free hemoglobin (SFHb) 3.4 g% and sufficient haptoglobin to bind 20 mg hemoglobin (HbBC=10 mg/ml) (equivalent to approximately 66.7 mg of protein).

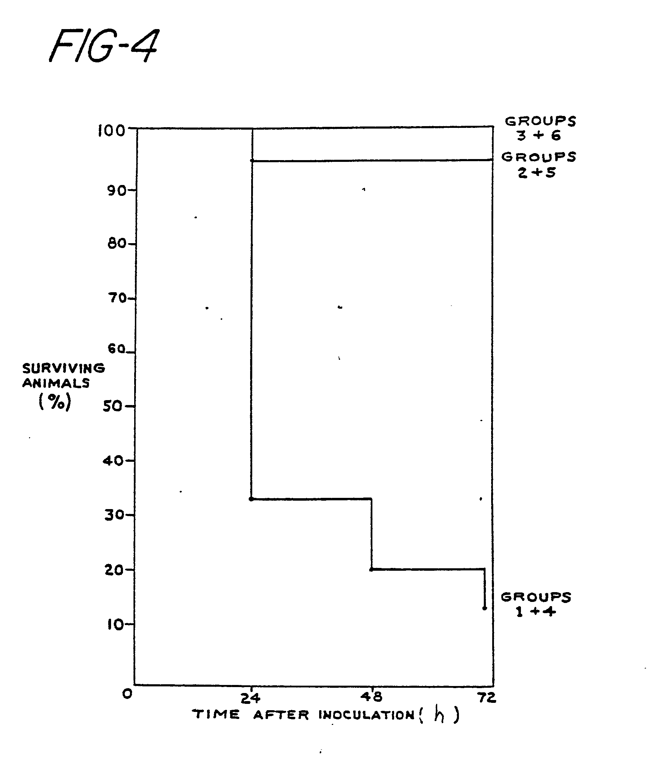

All of the animal subjects were given intraperitomeal injections that contained hemoglobin and haptoglobin in premixture. After administration of the injections, the rats were placed under study for a period of 72 hours, and mortality of the rats was observed at 24 hour intervals. The results of this experiment are set forth in Tables I and II, and are graphically presented in Figure 4.

TABLE I
Rat mortality after 48 hours

| | Mortality | |
| Group No. | No. dead | Total number tested |
|---|---|---|
| 1 | 6 | 7 |
| 2 | 1 | 7 |
| 3 | 0 | 7 |
| 4 | 6 | 8 |
| 5 | 0 | 8 |
| 6 | 0 | 8 |

TABLE II
Summary—Table I data

| | Mortality | |
| Inoculum | No. dead | Total No. tested |
|---|---|---|
| E. coli alone | 1 | 15 |
| E. coli plus hemoglobin | 12 | 15 |
| E. coli, hemoglobin plus haptoglobin | 0 | 15 |

A review of Figure 4 graphically confirms the results set forth in Table II, above. The rats inoculated with either the bacteria alone or the bacteria containing both hemoglobin and haptoglobin, were generally successfully resistent to fatal infection; of a total number of 30 rats tests in these two groups, only 1 rat administered E. coli alone died.

By contrast, the rats to which the inoculum including E. coli and hemoglobin was administered, suffered heavy mortality, with the heaviest death rate occurring within the first 24 hours after inoculation. During this first period, 10 out of the 15 rats died; after 48 hours 12 out of the 15 rats had died, while after 72 hours a total of 13 rats were dead. These tests further confirm the efficacy of haptoglobin in neutralising the growth accelerating effect of hemoglobin in contact with bacteria in the peritoneal environment.

Example IV

A large amount of growth media was prepared having a low iron content. The growth media comprised 5 milliliter aliquot portions of heat-inactivated human serum. Media were prepared with variant additives, and thereafter equal quantities of the bacteria were added. The media were thereafter inoculated with equal quantities of the bacteria. For primary groupings of inocula were developed as follows:

1. Group 1—The bacteria *E. coli* alone.

2. Group 2—*E. coli*, Ferric ammonium citrate (FAC) (amount of 45.3 µg), with or without 50 mg Cab-O-Sil® colloidal silica.

3. Group 3—*E. coli* and a small amount of hemoglobin (2 mg).

4. Group 4—*E. coli*, hemoglobin as in Group 3, and Cab-O-Sil® colloidal silica (50 mg).

In the instance where Cab-O-Sil® colloidal silica and hemoglobin were both added, a pre-mixture of the two was made, so that the hemoglobin was initially absorbed to the Cab-O-Sil® colloidal silica and the resulting adduct was then added to the growth medium.

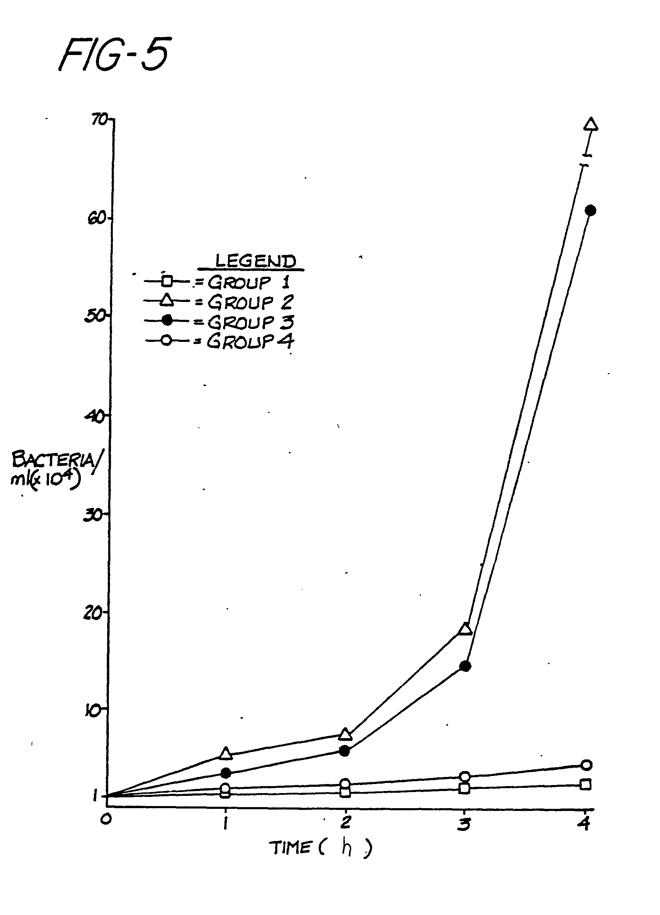

After preparation, all samples were maintained at the same temperature, and were observed for a period of time ranging up to about 24 hours following inoculation. Hourly bacterial counts were made, and have been provided, as to the first 4 hours of obervation, in Figure 5.

The data from the respective groupings is identified by the corresponding graphical symbols set forth in the legend. The data points plotted in Figure 5 represent the respective mean and standard deviation values determined from a comparison of 3 samples of each grouping observed and counted at the indicated time interval.

Referring to Figure 5, the rate of growth of the bacteria *E. coli* alone can be seen to be relatively flat, so that the initial or lag phase of bacterial growth is apparent. By contrast the addition of either the Ferric ammonium citrate (Group 2) of the hemoglobin alone (Group 3) to the growth medium results in a dramatic acceleration in bacterial growth. It is also noteworthy that Cab-O-Sil® colloidal silica when added to the Group 2 media, appeared to have no inhibitory effect upon bacterial growth, so that the availability of iron from this synthetic source was not controlled.

In Group 4, however, where both hemoglobin and Cab-O-Sil® colloidal silica were added, the acceleration of bacterial growth evident with Groups 2 and 3 was absent and had apparently been neutralised. Thus, Group 4 exhibited a growth pattern similar in rate to that of Group 1, comprising the low-iron containing serum alone.

The specificity of Cab-O-Sil® colloidal silica is suggested by a comparison of Groups 2 and 4. While Cao-O-Sil® colloidal silica appears to be ineffective against the synthetic iron-providing compound ferric ammonium citrate, activity is present with respect to hemoglobin.

Example V

Additional tests were conducted, similar in format to those described in Example IV above. Accordingly, similar growth media were prepared and were inoculated with *E. coli* in like fashion. Four primary groupings of additive inocula were developed as follows:

1. Group 1—The bacteria *E. coli* alone.

2. Group 2—*E. coli* and a small amount of hemoglobin (2 mg).

3. Group 3—*E. coli* and Cab-O-Sil® colloidal silica (50 mg).

4. Group 4—*E. coli*, hemoglobin as in Group 2 and Cab-O-Sil® colloidal silica as in Group 3.

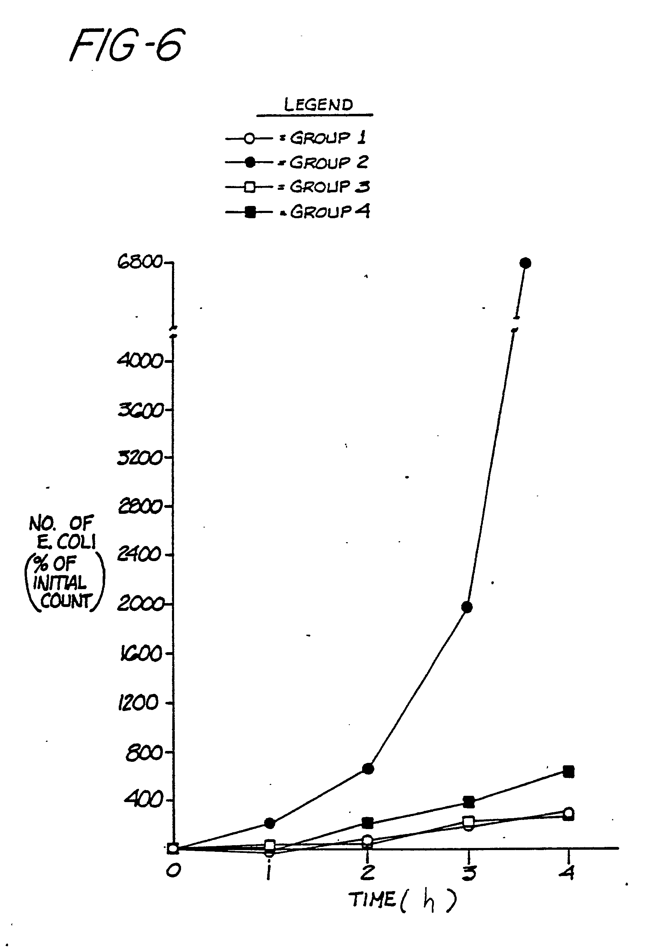

Similar conditions were observed, and the respective media were followed for approximately 24 hours, with the results of the first 4 hours of growth tabulated and set forth in Figure 6.

In similar fashion to the results of Figure 5, the media wherein the *E. coli* was present alone (Group 1) or was present in combination with Cab-O-Sil® colloidal silica, the growth exhibited followed the expected lag phase pattern. By contrast, growth was greatly accelerated in the instance where hemoglobin was added (Group 2) but was inhibited in the instance where Cab-O-Sil® colloidal silica was added to hemoglobin (Group 4). This last grouping exhibited a growth pattern comparable to that of Groups 1 and 3, where hemoglobin had not been added.

The results of these tests confirm the conclusion reached with respect to the tests depicted in Figure 5, that Cab-O-Sil® colloidal silica exhibits a specific effectiveness in the sequestration of hemoglobin.

Example VI

A specific preparation of a composite useful for coating or adsorption on solid substrates was prepared. A quantity of Cab-O-Sil® Grade M-5 colloidal silica and a polyisobutylene identified as Oppanol® Grade B-150 were combined in the ratio of 85% Cab-O-Sil® colloidal silica to 15% polyisobutylene. A dilute hydrocarbon solution of Oppanol® B-150 was prepared and the Cab-O-Sil® colloidal silica was suspended therein. The suspension was thereafter cast on a flat surface and dried, and yielded a flake-like composite in which the Cab-O-Sil® particles were bound to the polyisobutylene matrix.

Accordingly, the present invention includes coated articles useful to dispense the blood or hemoglobin-immobilising agents. For example, fibrous materials such as rayon, cellulose fiber and others utilised in the preparation of products such as gauze pads and skin bandages, may be impregnated or otherwise coated with the agents disposed in a solution or suspension, after which the resulting coating-article may be appropriately dried to volatize off any undesired vehicle. In the instance where the suspension of Cab-O-Sil® particles is prepared as set forther in Example VI, above, the intended suspension prior to the evaporation of the solvent, so that the resulting particles or flakes may be fixed to the substrate when dry.

As mentioned earlier, the present invention carries a broad spectrum of uses, going beyond the treatment of peritoneal infection. The use of the agents used in the invention may extend to topical treatments and other personal hygiene instances where a fluid and a bacteria are in contact with each other, and where that fluid may contain a quantity of blood.

## Claims

1. The use of agents capable of aggregating red blood cells and/or hemoglobin selected from the complexing proteins haptoglobin; hemopexin; transferrin; a mixture of haptoglobin, hemopexin and transferrin; plant lectins specific for sugars, and mixtures thereof, colloidal silica, anion exchange resins, resins capable of binding compounds having available sulfhydryl groups, a composite of colloidal silica and polyolefinic elastomer, and mixtures thereof for the manufacture of a medicament for the treatment of bacterial infections.

2. The use according to Claim 1 wherein the agent comprises an anion exchange resin selected from diethylaminoethylcellulose, guanidoethylcellulose, diethyl (2 - hydroxypropyl) aminoethyl dextran; polyethyleneimine or the composite of colloidal silica and polyisobutylene.

3. The use according to Claim 1 wherein the agent comprises a mixture of the anion exchange resin diethylaminoethylcellulose and colloidal silica.

4. The use according to Claim 1 wherein said agent comprises said composite alone.

5. The use according to Claim 2 or 4 wherein said composite contains 85% colloidal silica and 15% polyisobutylene.

6. The use according to any preceding claim wherein the agent is coated on at least a portion of the effective surface area of an absorbent substrate.

7. The use according to Claim 6 wherein said absorbent substrate is selected from surgical sponges, bandages, gauze, sanitary napkins and tampons.

## Patentansprüche

1. Die Verwendung von Mitteln, die fähig sind rote Blutzellen und/oder Hämoglobin zu aggregieren, ausgewählt aus:

den komplexierenden Proteinen Haptoglobin, Hämopexin, Transferrin, einer Mischung von Haptoglobin, Hämopexin und Transferrin, für Zucker spezifischen Pflanzenlektinen, und Mischungen davon;

kolloidalem Kieselgel, Anionenaustauschharzen, Harzen, die Verbindungen mit verfügbaren Sulfhydrylgruppen binden können, einer Zusammensetzung aus kolloidalem Kieselgel und polyolefinischem Elastomer, und Mischungen davon;

für die Herstellung eines Medikamentes zur Behandlung von bakteriellen Infektionen.

2. Die Verwendung nach Anspruch 1, wobei das Mittel ein Anionenaustauschharz umfaßt, ausgewählt aus:

Diethylaminoethylcellulose, Guanidoethylcellulose, Diethyl(2 - hydroxypropyl)aminoethyldextran;

Polyethylenimin oder der Zusammensetzung von kolloidalem Kieselgel und Polyisobutylen.

3. Die Verwendung nach Anspruch 1, wobei das Mittel eine Mischung des Anionenaustauschharzes Diethylaminoethylcellulose und kolloidalem Kieselgel umfaßt.

4. Die Verwendung nach Anspruch 1, wobei das Mittel die Zusammensetzung alleine umfaßt.

5. Die Verwendung nach Anspruch 2 oder 4, wobei die Zusammensetzung 85% kolloidales Kieselgel und 15% Polyisobutylen enthält.

6. Die Verwendung nach einem der vorhergehenden Ansprüche, wobei das Mittel wenigstens auf einen Teil der wirksamen Oberflächenfläche eines absorbierenden Substrates als Schicht aufgetragen ist.

7. Die Verwendung nach Anspruch 6, wobei das absorbierende Substrat ausgewählt wird aus:

chirurgischen Schwämmen, Bandagen, Gazen, Damenbinden und Tampons.

## Revendications

1. Utilisation d'agents capables d'agréger les globules rouges et/ou l'hémoglobine choisis dans les protéines complexantes haptoglobine, hemopexine, transferrine; un mélange d'haptoglobine, d'hemopexine et de transferrine; des lectines de plantes spécifiques aux sucres, et leurs mélanges, de la silice colloïdale, des résines d'échange d'anions, des résines capables d'agréger des composés ayant des groupes sulphydryls disponibles, un composé de silice colloïdale et d'élastomère polyoléfinique, et leurs mélanges pour la fabrication d'un médicament pour le traitement d'infections bactériennes.

2. Utilisation selon la revendication 1, dans laquelle l'agent contient une résine d'échange d'anions choisie parmi la diéthylaminoéthylcellulose, la guanidoéthylcellulose, le diéthyl (2 - hydroxypropyl) aminoéthyl dextrane, le polyéthylèneimine ou le composé de silice colloïdale et de polyisobutylène.

3. Utilisation selon la revendication 1, dans laquelle l'agent contient un mélange de résine d'échange d'anions, de diéthylaminoéthylcellulose et de silice colloïdale.

4. Utilisation selon la revendication 1, dans laquelle ledit agent contient ledit composé seul.

5. Utilisation selon la revendication 2 ou 4, dans laquelle ledit composé contient 85% de silice colloïdale et 15% de polyisobutilène.

6. Utilisation selon l'une quelconque des revendications précédentes, dans laquelle ledit agent est recouvert sur au moins une partie de la surface efficace d'un substrat absorbatn.

7. Utilisation selon la revendication 6, dans laquelle ledit substrat absorbant est choisi dans les éponges chirurgicales, les bandages, la gaze, les serviettes et les tampons hygiéniques.

# FIG-1

FIG-2

600 —

LEGEND
—○— = GROUP 1
—●— = GROUP 2
—△— = GROUP 3
—□— = GROUP 4

500 —

NO. OF E.COLI
(% OF ZERO
TIME)

400 —

300 —

200 —

100 —

0 —

1    2    3    4    5

t ( h )

FIG-3

Legend:
- ● = GROUP 1
- □ = GROUP 2
- ○ = GROUP 3

Y-axis: NO. OF ANIMALS SURVIVING (%), 100% to 0

X-axis: TIME ( h ), 24, 48, 72

EP 0 077 826 B1

# FIG-4

EP 0 077 826 B1

FIG-5

# FIG-6

LEGEND

—○— = GROUP 1
—●— = GROUP 2
—□— = GROUP 3
—■— = GROUP 4

NO. OF
E. COLI
(% OF
INITIAL
COUNT)

TIME ( h )

6